# EUROPÄISCHE PATENTSCHRIFT

(11) **EP 4 311 525 B1**
(45) Veröffentlichungstag und Bekanntmachung des Hinweises auf die Patenterteilung: **03.09.2025**
(21) Anmeldenummer: 23184058.8
(22) Anmeldetag: 07.07.2023
(51) Int. Cl.: A61F 2/46, A61F 2/38, A61F 2/30

(54) **CHIRURGISCHES INSTRUMENT, FEMORALES PROBEIMPLANTAT UND CHIRURGISCHES INSTRUMENTENSYSTEM**
SURGICAL INSTRUMENT, TRIAL FEMORAL IMPLANT AND INSTRUMENT SYSTEM
INSTRUMENT CHIRURGICAL, IMPLANT D'ÉCHANTILLON FÉMORAL ET SYSTÈME D'INSTRUMENT CHIRURGICAL

(30) Priorität: 25.07.2022 DE 102022207577
(43) Veröffentlichungstag der Anmeldung: 31.01.2024
(73) Patentinhaber: Aesculap AG, 78532 Tuttlingen (DE)
(72) Erfinder: Firmbach, Franz-Peter, 78576 Emmingen-Liptingen (DE); Stoffels, Lilli, 75365 Calw (DE)
(74) Vertreter: Patentanwälte Ruff, Wilhelm, Beier, Dauster & Partner mbB

(56) Entgegenhaltungen:
- WO-A1-2012/078498
- WO-A1-2022/090312
- CN-A- 114 271 894
- US-A1- 2019 150 948
- US-A1- 2020 113 710

## Beschreibung

Die Erfindung betrifft ein chirurgisches Instrument mit den oberbegrifflichen Merkmalen des Anspruchs 1. Die Erfindung betrifft außerdem ein femorales Probeimplantat mit den oberbegrifflichen Merkmalen des Anspruchs 7. Ferner betrifft die Erfindung ein chirurgisches Instrumentensystem zur Verwendung bei einer Kniegelenkersatzoperation.

Bei einer Kniegelenkersatzoperation (englisch: Total Knee Arthroplasty (TKA)) werden abgenutzte oder anderweitig durch Krankheit oder einen Unfall beeinträchtigte Gelenkflächen des Femurs oder/und der Tibia durch künstliche Gelenkflächen einer Kniegelenkprothese ersetzt. Solche Kniegelenkprothesen umfassen üblicherweise eine Femurkomponente und eine Tibiakomponente. Die Femurkomponente wird am distalen Ende des Femurs implantiert. Die Tibiakomponente wird am proximalen Ende der Tibia implantiert.

Vor der Implantation der prothetischen Komponenten werden der distale Femur und die proximale Tibia reseziert. Hierfür bringt der Chirurg unterschiedliche Resektionsschnitte an und trennt Knochen- oder/und Knorpelmaterial von dem jeweiligen Knochen ab. Durch die Resektion wird der jeweilige Knochen in seiner Form an die aufzunehmende prothetische Komponente angepasst.

Die Resektion kann auf der Grundlage unterschiedlicher Konzepte ausgeführt werden. Ein Konzept zielt darauf ab, die Spannungen der Bänder des Knies bei der Gelenkbewegung ausgeglichen zu halten. Hierdurch soll eine bessere Funktion der Kniegelenkprothese gewährleistet werden. Dieses Konzept wird allgemein als "gap balancing" bezeichnet. Bei anderen Konzepten entfernt der Chirurg mittels der Resektion eine bestimmte Menge an Knochen- oder/und Knorpelmaterial. Solche Konzepte werden allgemein als "measured resection" bezeichnet. Die Ausrichtung der Resektionsschnitte in Bezug auf die Anatomie des Patienten bestimmt die spätere Ausrichtung der implantierten Komponenten und folglich auch die Orientierung der prothetischen Gelenkachsen. Die Ausrichtung der Resektionsschnitte ist daher von besonderer Bedeutung.

Bei der Ausrichtung der Resektionsschnitte werden in erster Linie drei Ansätze unterschieden: mechanisch, anatomisch und kinematisch. Bei der mechanischen Ausrichtung wird die proximale Tibia senkrecht zur Längsachse des Tibiaschafts reseziert. Die Resektion des distalen Femurs erfolgt entsprechend hieran angepasst. Erforderlichenfalls werden Bandentlastungen (englisch: ligament releases) durchgeführt. Bei der anatomischen Ausrichtung wird versucht, die Tibia in einem Varus-Winkel von 3° zu resezieren. Die Femurresektion und Bandentlastungen werden mit dem Ziel einer geraden Hüft-Knie-Knöchel-Achse des Beins durchgeführt. Ziel der kinematischen Ausrichtung (englisch: kinematic alignment, nachfolgend abgekürzt als KA) ist es, die künstlichen Gelenkflächen der prothetischen Komponenten auf dem Niveau der präarthritischen, defektfreien natürlichen Gelenkflächen zu implantieren.

Bei einem KA erfolgt die Ausrichtung der Resektionsschnitte oftmals ausgehend von dem distalen Femur. Die Resektion der proximalen Tibia erfolgt hieran angepasst. Zum Zweck der zeitlich nacheinander erfolgenden Resektion von Femur und Tibia sind spezielle chirurgische Instrumente bekannt, die auch als tibiales Ausricht- oder/und Übertragungswerkzeug (tibial cut alignment guide) bezeichnet werden. Solche Instrumente erlauben eine Übertragung der Ausrichtung der femoralen Resektionsschnitte auf die Tibia. Die Übertragung erfolgt üblicherweise nach einer wenigstens distalen Resektion des Femurs, bei welcher die distalen Kondylen abgetrennt werden. Die Übertragung kann in Extension oder Flexion erfolgen. Bei einer Variante der KA wird zunächst der distale Femur vollständig präpariert (all femur first). Dabei werden die am distalen Femur vorzunehmenden Resektionsschnitte an der Anatomie des Patienten ausgerichtet und am distalen Femur angebracht. Anschließend wird ein femorales Probeimplantat an dem distalen Femur angebracht. Das bereits nach der Anatomie des Patienten ausgerichtete femorale Probeimplantat wird dann als Referenzkomponente herangezogen, deren Ausrichtung mittels des Übertragungswerkzeugs auf die proximale Tibia übertragen wird, um die daran vorzunehmenden Resektionsschnitte auszurichten und anzubringen. Es versteht sich, dass alternativ ein Vorgehen in umgekehrter Reihenfolge denkbar ist, also von der proximalen Tibia ausgehend.

Besagte Übertragungswerkzeuge weisen üblicherweise eine Befestigungseinrichtung auf, die zur lösbaren Befestigung an einem femoralen Probeimplantat eingerichtet ist. Typischerweise ist die Befestigungseinrichtung formschlüssig lösbar an dem Probeimplantat befestigbar. Solche Probeimplantate sind oftmals dünnwandig ausgebildet. Für die Ausbildung des Formschlusses steht daher nur eine relativ geringe Materialstärke zur Verfügung. Die Ausbildung des Formschlusses und seine zuverlässige Aufrechterhaltung erweisen sich folglich als besonders herausfordernd. Infolge der oftmals geringen Materialstärke des Probeimplantats kann es zu elastischen oder/und plastischen Verformungen kommen. Diese können zu einem Lockern oder/und Lösen des Formschlusses führen. Zudem können Verkippbewegungen der Befestigungseinrichtung relativ zu dem Probeimplantat auftreten. Hierdurch kann die Ausrichtung des Übertragungswerkzeugs verfälscht werden.

Aus der CN 114 271 894 A sind ein chirurgisches Instrument und ein femorales Probeimplantat mit den oberbegrifflichen Merkmalen der Ansprüche 1 und 7 bekannt. Bei dem bekannten chirurgischen Instrument sind die Positionierstifte jeweils relativ zu der zugehörigen Aufnahmebohrung drehbeweglich und weisen endseitige Exenterabschnitte auf, die mittels einer Drehbewegung der Positionierstifte an einem Hinterschnitt der jeweiligen Aufnahmebohrung festlegbar sind.

Aus der US 2019/150948 A und der WO 2012/078498 A1 sind weitere chirurgische Instrumente und Probeimplantate bekannt. Die dort gezeigten Probeimplantate weisen jeweils proximodistal längserstreckte Aufnahmebohrungen auf, die zur Aufnahme von Befestigungsstiften des jeweiligen chirurgischen Instruments eingerichtet sind.

Aus der US 2020/113710 A1 ist ein chirurgisches Instrument bekannt, das zum Extrahieren von Implantaten eingerichtet ist und Befestigungsstifte aufweist, die mit Aufnahmebohrungen des zu entfernenden Implantats in Eingriff gebracht werden können.

Aufgabe der Erfindung ist es, ein chirurgisches Instrument sowie ein femorales Probeimplantat und ein chirurgisches Instrumentensystem bereitzustellen, welche Vorteile gegenüber herkömmlichen chirurgischen Instrumenten sowie femoralen Probeimplantaten und chirurgischen Instrumentensystemen bieten. Insbesondere soll eine besonders verkippsichere Befestigung des chirurgischen Instruments an dem Probeimplantat ermöglicht werden.

Diese Aufgabe wird durch das Bereitstellen eines chirurgischen Instruments mit den Merkmalen des Anspruchs 1, eines femoralen Probeimplantats mit den Merkmalen des Anspruchs 7 sowie eines chirurgischen Instrumentensystems mit den Merkmalen des Anspruchs 12 gelöst. Bevorzugte Ausführungsformen sind Gegenstand der abhängigen Patentansprüche.

Das erfindungsgemäße chirurgische Instrument dient zur Verwendung bei einer Kniegelenkersatzoperation und weist auf: eine Befestigungseinrichtung, welche zur lösbaren Befestigung an einem an einem distalen Femur angebrachten femoralen Probeimplantat eingerichtet ist, wobei die Befestigungseinrichtung zwei mediolateral voneinander beabstandet angeordnete Positionierstifte aufweist, die zur Positionierung der Befestigungseinrichtung in zwei zu den Positionierstiften komplementären Aufnahmebohrungen des Probeimplantats in einer posterioren Richtung einsteckbar ausgebildet sind, wobei die Befestigungseinrichtung wenigstens ein Fixierelement zum formschlüssigen Fixieren der Befestigungseinrichtung an dem Probeimplantat in einer anterioren Richtung aufweist. Das Probeimplantat ist nicht Bestandteil des chirurgischen Instruments. Durch Zusammenwirken der Positionierstifte mit den Aufnahmebohrungen lässt sich das chirurgische Instrument mediolateral, anteroposterior und proximodistal in korrekter Ausrichtung relativ zum Probeimplantat positionieren. Mittels des Fixierelements lässt sich die Positionierung des chirurgischen Instruments formschlüssig fixieren. Das Fixierelement wirkt einem ungewollten Lösen der Steckverbindung entgegen. Im Speziellen wird einem anterioren Herausgleiten der Positionierstifte aus den Aufnahmebohrungen entgegengewirkt. Das Fixierelement sorgt in vorteilhafter Weise dafür, dass die Positionierstifte über eine ausreichende Tiefe in den Aufnahmebohrungen aufgenommen bleiben. Eine zwischen den Positionierstiften und den Aufnahmebohrungen auftretende Flächenpressung kann folglich geringgehalten werden. Entsprechend gering kann auch eine Verformung der Positionierstifte oder/und der Aufnahmebohrungen ausfallen, wenn zwischen der Befestigungseinrichtung und dem Probeimplantat ein verkippendes Drehmoment wirkt. Einem unerwünschten Verkippen des chirurgischen Instruments relativ zu dem Probeimplantat infolge wird somit besonders wirksam entgegengewirkt. Entsprechend erweist sich das chirurgische Instrument als besonders zuverlässig und robust in der Anwendung. Zudem ermöglicht es eine besonders präzise Übertragung der Ausrichtung des Probeimplantats auf eine proximale Tibia.

Die in dieser Beschreibung verwendeten Lage- und Richtungsbezeichnungen beziehen sich auf den Körper eines Patienten, insbesondere dessen Femur, und sind insoweit gemäß ihrer üblichen anatomischen Bedeutung zu verstehen. Folglich bedeutet "anterior" vordere(r) oder vorne liegend, "posterior" hintere(r) oder hinten liegend, "medial" innere(r) oder innen liegend, "lateral" äußere(r) oder außen liegend, "proximal" zum Körperzentrum hin und "distal" vom Körperzentrum entfernt. Weiter bedeuten "proximodistal" entlang, vorzugsweise parallel zu, einer proximal-distal ausgerichteten Achse, "anteroposterior" entlang, vorzugsweise parallel zu, einer anterior-posterior ausgerichteten Achse und "mediolateral" entlang, vorzugsweise parallel zu, einer medial-lateral ausgerichteten Achse. Die besagten Achsen sind orthogonal zueinander ausgerichtet und können selbstverständlich in Bezug zu nicht mit der Anatomie des Patienten in Verbindung stehenden X-, Y- und Z-Achsen gesetzt werden. Beispielsweise kann die proximaldistale Achse alternativ als X-Achse bezeichnet werden. Die medial-laterale Achse kann als Y-Achse bezeichnet werden. Die anterior-posteriore Achse kann als Z-Achse bezeichnet werden. Zur verbesserten Veranschaulichung und der Einfachheit der Bezeichnungen wegen werden nachfolgend in erster Linie die besagten anatomischen Lage- und Richtungsbezeichnungen verwendet.

In Ausgestaltung der Erfindung weist das Fixierelement wenigstens einen Formschlussabschnitt zur Ausbildung eines Formschlusses zwischen Befestigungseinrichtung und Probeimplantat auf. Der Formschlussabschnitt kann eine Nase, einen Haken, einen Stift, eine Schraube oder Ähnliches umfassen. Dies erlaubt eine besonders zuverlässige Fixierung des chirurgischen Instruments an dem femoralen Probeimplantat.

In weiterer Ausgestaltung der Erfindung umfasst das Fixierelement wenigstens einen Schnapphaken. Ein solcher Schnapphaken kann beim Einführen der Positionierstifte in die komplementären Aufnahmebohrungen selbsttätig an dem Probeimplantat einrasten. Somit kann vorteilhaft auf eine gesonderte Verriegelungsmaßnahme zur Ausbildung des Formschlusses beim Montieren des chirurgischen Instruments an dem femoralen Probeimplantat verzichtet werden. Entsprechend erweist sich das chirurgische Instrument als besonders einfach in der Handhabung. Zudem hilft es eine Operationsdauer kurz zu halten.

Weiter gemäß der Erfindung ist das Fixierelement relativ zu den Positionierstiften zwischen einer Fixier- und einer Freigabeposition verstellbeweglich. In der Fixierposition kann der Formschluss ausgebildet sein. In der Freigabeposition kann der Formschluss gelöst sein. Vorzugsweise lässt sich das Fixierelement manuell zwischen seiner Freigabeposition und seiner Fixierposition bewegen. Somit kann der Formschluss durch Verstellen des Fixierelements in seine Freigabeposition erforderlichenfalls gelöst werden, um das chirurgische Instrument vom Probeimplantat zu trennen. Das Lösen des Formschlusses ist vorzugsweise rein manuell oder/und ohne eine Verwendung eines gesonderten Werkzeugs möglich.

In weiterer Ausgestaltung der Erfindung umfasst die Befestigungseinrichtung wenigstens ein Federelement, insbesondere ein Torsions- oder Balkenfederelement, zum Erzeugen einer Federkraft, die das Fixierelement in Richtung seiner Fixierposition zwingt. Ein Verstellen des Fixierelements in seine Freigabeposition kann also entgegen und unter Überwindung der Federkraft erfolgen. Somit lässt sich besonders wirksam vermeiden, dass der Formschluss versehentlich gelöst wird. Außerdem kann das Fixierelement durch die Federkraft selbsttätig in seine Fixierposition verstellt und dort gehalten werden. Der Formschluss kann also selbsttätig ausgebildet werden, wenn die Positionierstifte in die Aufnahmebohrungen eingeführt werden. Eine gesonderte Betätigung des Fixierelements durch den Operateur ist in diesem Fall obsolet.

Weiter gemäß der Erfindung sind die Positionierstifte jeweils zwischen einem ersten und einem zweiten Ende anteroposterior längserstreckt, wobei ein jeweils an dem ersten Ende vorhandener erster Endabschnitt in einer zugehörigen der Aufnahmebohrungen des Probeimplantats unter Ausbildung einer Steckverbindung in posteriorer Richtung aufnehmbar ist. Derartige Positionierstifte sind, insbesondere mit ausreichender Präzision, besonders kostengünstig herstellbar.

In weiterer Ausgestaltung der Erfindung bildet der Formschlussabschnitt eine Hinterschneidung bezüglich der anterioren Richtung aus. Die Hinterschneidung verhindert ein unbeabsichtigtes Trennen der Befestigungseinrichtung vom Probeimplantat in anteriorer Richtung. Dies erlaubt eine besonders zuverlässiges Fixieren der Befestigungseinrichtung.

In weiterer Ausgestaltung der Erfindung liegen die ersten Enden posterior frei und die Hinterschneidung liegt anterior frei, so dass das Probeimplantat zwischen den ersten Enden einerseits und der Hinterschneidung andererseits anteroposterior positionierbar, insbesondere einspannbar, ist. Mit anderen Worten: die ersten Enden und die Hinterschneidung sind anteroposterior zueinander entgegengesetzt orientiert. Somit lässt sich vorteilhaft die Position der Befestigungseinrichtung sowohl anterior als auch posterior fixieren.

Das erfindungsgemäße femorale Probeimplantat ist zur Verwendung bei einer Kniegelenkersatzoperation vorgesehen und weist auf: eine Knochenkontaktfläche, die zur Kontaktierung eines distalen Femurs eingerichtet ist, zwei Aufnahmebohrungen, die jeweils zur Aufnahme eines Positionierstifts eines erfindungsgemäßen und der voranstehenden Beschreibung entsprechenden, chirurgischen Instruments eingerichtet und anteroposterior längserstreckt sind, und einen Fixierabschnitt, welcher zur formschlüssigen Verbindung mit einem Fixierelement des chirurgischen Instruments eingerichtet ist. Die oben genannten Vorteile des erfindungsgemäßen chirurgischen Instruments übertragen sich mutatis mutandis auch auf das erfindungsgemäße femorale Probeimplantat.

Weiter gemäß der Erfindung ist die Knochenkontaktfläche an einer proximalen Rückseite eines Grundkörpers des Probeimplantats angeordnet und liegt einer distalen Vorderseite des Grundkörpers gegenüber, wobei der Grundkörper zwei mediolateral zueinander beabstandete und jeweils anteroposterior erstreckte Kondylenabschnitte aufweist, die an ihren anterioren Enden in einen Verbindungsabschnitt des Grundkörpers übergehen, wobei der Fixierabschnitt an einem posterioren Ende des Verbindungsabschnitts mediolateral zwischen den beiden Kondylenabschnitten angeordnet ist. Hieraus ergibt sich eine zentrale Anordnung des Fixierabschnitts. Der Fixierabschnitt kann dabei in einem Bereich angeordnet sein, in welchem vor der Resektion des Femurs die Kreuzbandhöhle (englisch: intercondylar notch) vorhanden war. Der so angeordnete Fixierabschnitt erlaubt eine zentrale Einleitung einer Haltekraft vom Fixierelement in das Probeimplantat. Dies sorgt für eine besonders stabile und verkippsichere lösbare Verbindung von Befestigungseinrichtung und Probeimplantat.

In weiterer Ausgestaltung der Erfindung ist der Fixierabschnitt mediolateral zwischen den beiden Aufnahmebohrungen sowie - alternativ oder zusätzlich - proximodistal im Abstand zu den beiden Aufnahmebohrungen angeordnet. Die mediolaterale Anordnung sorgt für eine besonders gleichmäßige Einleitung von Befestigungskräften über das Formschlusselement und die Positionierstifte in das Probeimplantat. Der proximodistale Abstand zwischen dem Fixierelement und den Aufnahmebohrungen kann vorteilhaft als Hebel zum Abstützen von Drehmomenten zwischen Befestigungseinrichtung und Probeimplantat fungieren.

In weiterer Ausgestaltung der Erfindung ist wenigstens eine, vorzugsweise jede, der Aufnahmebohrungen als Sackloch ausgebildet. Ein Boden der sacklochförmigen Aufnahmebohrungen bildet dabei vorteilhaft einen, insbesondere posterioren, Anschlag für die Positionierstifte. Entsprechend lässt sich eine posteriore Position des chirurgischen Instruments relativ zum Probeimplantat auf besonders einfache Weise und dauerhaft festlegen.

Die Erfindung betrifft ferner ein chirurgisches Instrumentensystem zur Verwendung bei einer Kniegelenkersatzoperation, welches aufweist: ein erfindungsgemäßes chirurgisches Instrument gemäß der vorhergehenden Beschreibung, ein erfindungsgemäßes femorales Probeimplantat gemäß der vorhergehenden Beschreibung, wobei das chirurgische Instrument lösbar an dem Probeimplantat befestigt ist, wobei die Positionierstifte und die Aufnahmebohrungen komplementär zueinander ausgebildet und die Positionierstifte entlang der posterioren Richtung wenigstens teilweise in die Aufnahmebohrung gesteckt sind, wobei das Fixierelement und der Fixierabschnitt zum Ausbilden eines Formschlusses aufeinander abgestimmt sind und das chirurgische Instrument an dem Probeimplantat mittels des Formschlusses, insbesondere in anteriorer Richtung, fixiert ist. Die voranstehend aufgezeigten Vorteile des erfindungsgemäßen chirurgischen Instruments sowie des erfindungsgemäßen femoralen Probeimplantats übertragen sich mutatis mutandis auch auf das erfindungsgemäße chirurgische Instrumentensystem gemäß der Erfindung.

In weiterer Ausgestaltung der Erfindung umfasst das chirurgische Instrumentensystem mehrere Probeimplantate unterschiedlicher Größen, wobei bei allen Probeimplantaten beide der Aufnahmebohrungen, insbesondere bezüglich ihrer Mittellängsachsen, in einem identischen proximodistalen Abstand zu einem proximal äußersten Punkt des jeweiligen Probeimplantats angeordnet sind, wobei die Aufnahmebohrungen jedes der Probeimplantate in einem identischen mediolateralen Abstand, insbesondere bezüglich der Mittellängsachsen, voneinander angeordnet sind. Gemeint ist damit ausdrücklich nicht, dass der proximodistale und der mediolaterale Abstand bei ein und demselben Probeimplantat betragsmäßig identisch sein müssen. Vielmehr ist den mehreren unterschiedlich großen Probeimplantaten größenunabhängig sowohl der proximodistale Abstand wie auch der mediolaterale Abstand gemein. Mit anderen Worten: Alle Probeimplantate des chirurgischen Instrumentensystems weisen einen einheitlichen proximalen Abstand und einen einheitlichen mediolateralen Abstand auf. Vorteilhaft lässt sich somit ein- und dasselbe chirurgische Instrument mit verschieden großen Probeimplantaten verwenden. Die Größen der Probeimplantate können abgestimmt sein auf verschieden groß ausgebildete oder gewachsene Femurknochen. Entsprechend lässt sich ein Probeimplantat bestimmter Größe aus den mehreren Probeimplantaten auswählen, das am besten auf eine gegebene Anatomie des Patienten passt. Unabhängig von der Größe des Probeimplantats lässt sich mittels des chirurgischen Instruments die Ausrichtung des Probeimplantats an der Anatomie des Patienten auf die Tibia übertragen. Insbesondere ergibt sich unabhängig von der gewählten Größe des Probeimplantats eine korrekte sog. Tibiaschnitthöhe. Der proximal äußerste Punkt kann auch als "Dwell-Point" bezeichnet werden.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.

Es versteht sich, dass die voranstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.
- Fig. 1: zeigt eine schematische Perspektivdarstellung einer Ausführungsform eines erfindungsgemäßen chirurgischen Instrumentensystems mit einem gemäß der Erfindung ausgeführten chirurgischen Instrument in einer intraoperativen Situation,
- Fig. 2: ein Detail B des chirurgischen Instrumentensystems nach Fig. 1,
- Fig. 3: das chirurgische Instrumentensystem nach den Fig. 1 und 2 in einer schematischen Perspektivdarstellung aus einem anderen Blickwinkel,
- Fig. 4: eine schematische Vorderansicht des chirurgischen Instrumentensystems nach den Fig. 1 bis 3,
- Fig. 5: eine schematische Schnittdarstellung entlang einer Schnittlinie D-D nach Fig. 4,
- Fig. 6: ein Detail H der Darstellung nach Fig. 5,
- Fig. 7: eine schematische Schnittdarstellung entlang einer Schnittlinie G-G nach Fig. 4,
- Fig. 8: eine schematische Perspektivdarstellung einer weiteren Ausführungsform eines erfindungsgemäßen chirurgischen Instrumentensystems mit einem weiteren gemäß der Erfindung ausgeführten chirurgischen Instrument.

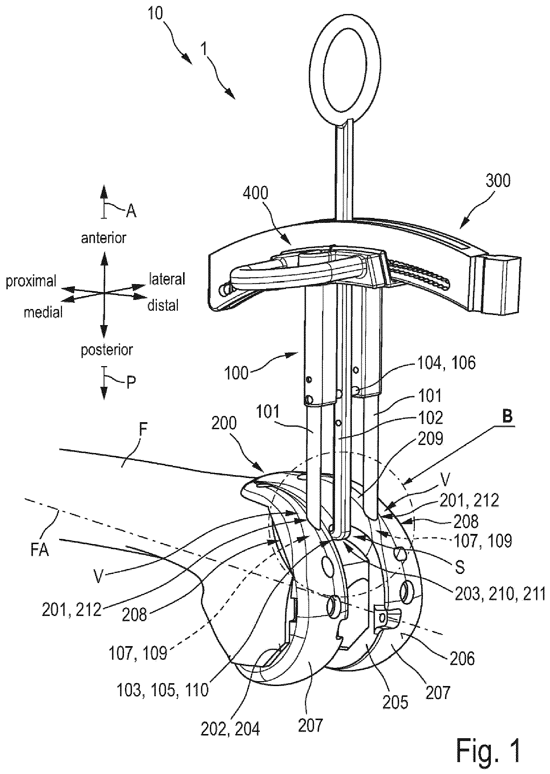

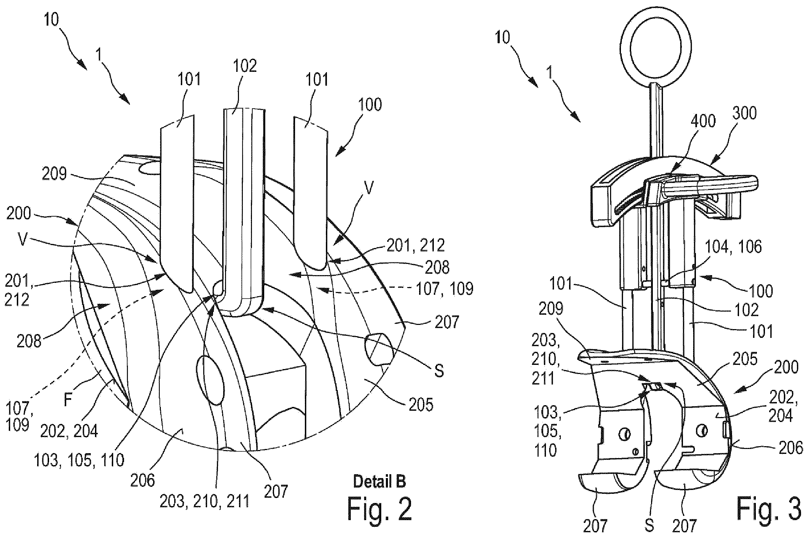

Gemäß den Fig. 1 und 2 ist ein chirurgisches Instrument 1 zur Verwendung bei einer Kniegelenkersatzoperation vorgesehen. Das chirurgische Instrument 1 kann auch als Übertragungswerkzeug oder tibia transfer tool bezeichnet werden und dient einer referenzierten Positionierung eines Tibiaschnittblocks an einer proximalen Tibia. Tibiaschnittblock und Tibia sind in den Figuren nicht gezeigt. Als Referenz für die Position des Tibiaschnittblocks dient ein bereits an einem distalen Femur F positioniertes femorales Probeimplantat 200. Das femorale Probeimplantat 200 und der Tibiaschnittblock sind nicht Bestandteil des chirurgischen Instruments 1. Das Probeimplantat 200 und das chirurgische Instrument 1 sind Bestandteil eines chirurgischen Instrumentensystems 10. Das chirurgische Instrument 1 und das chirurgische Instrumentensystem 10 eignen sich insbesondere für Kniegelenkersatzoperationen, die dem sogenannten all femur first-Ansatz folgen. Gemäß diesem Ansatz werden zunächst sämtliche erforderliche femorale Resektionsschnitte angebracht. Nach Resektion des distalen Femurs F wird das femorale Probeimplantat 200 befestigt, welches dann als femorale Referenzkomponente für das chirurgische Instrument 1 fungieren kann. In den Fig. 1 und 2 ist das femorale Probeimplantat 200 an dem zuvor resezierten distalen Femur F befestigt. Mittels des chirurgischen Instruments 1 lässt sich eine bereits an der Anatomie des Patienten vorgenommene Ausrichtung des Probeimplantats 200 auf eine proximale Tibia transferieren, um dort in korrekter Ausrichtung wenigstens einen Resektionsschnitt vorzunehmen.

Das chirurgische Instrument 1 weist eine Befestigungseinrichtung 100 auf. Die Befestigungseinrichtung 100 ist zur lösbaren Befestigung an dem femoralen Probeimplantat 200 eingerichtet. Die Befestigungseinrichtung 100 umfasst zwei Positionierstifte 101. Die beiden Positionierstifte 101 sind mediolateral voneinander beabstandet angeordnet. Die Positionierstifte 101 sind zur Positionierung der Befestigungseinrichtung 100 in zwei Aufnahmebohrungen 201 des Probeimplantats 200 einsteckbar ausgebildet. Die Aufnahmebohrungen 201 sind komplementär zu den Positionierstiften 101 ausgebildet. Die Positionierstifte 101 sind in einer posterioren Richtung P in die Aufnahmebohrungen 201 einsteckbar ausgebildet. Mit anderen Worten: Die Positionierstifte 101 können entlang der posterioren Richtung P in die komplementären Aufnahmebohrungen 201 eingeführt oder eingeschoben werden.

Die Befestigungseinrichtung 100 umfasst ferner wenigstens ein Fixierelement 102. Das Fixierelement 102 dient zum formschlüssigen Halten oder/und Fixieren der Befestigungseinrichtung 100 an dem Probeimplantat 200. Das Fixierelement 102 ist zum formschlüssigen Halten oder/und Fixieren der Befestigungseinrichtung 100 an dem Probeimplantat 200 in einer anterioren Richtung A eingerichtet. Das Fixierelement 102 weist wenigstens einen Formschlussabschnitt 103 auf. Der Formschlussabschnitt 103 dient zur Ausbildung eines Formschlusses S zwischen Befestigungseinrichtung 100 und Probeimplantat 200. Der Formschlussabschnitt 103 kann in Form einer Nase, eines Hakens, eines Stifts, einer Schraube oder Ähnlichem ausgebildet sein. Vorliegend umfasst das Fixierelement 102 einen Schnapphaken 105. Der Schnapphaken 105 rastet beim Einstecken der beiden Positionierstifte 101 in die Aufnahmebohrungen 201 selbsttätig an dem Probeimplantat 200 ein.

Das Fixierelement 102 ist relativ zu den Positionierstiften 101 zwischen einer Fixier- und einer Freigabeposition verstellbeweglich. In der Fixierposition kann der Formschluss S ausgebildet sein. In der Freigabeposition kann der Formschluss S aufgehoben oder gelöst sein. In der in den Fig. 1 und 2 gezeigten intraoperativen Situation befindet sich das Fixierelement 102 in seiner Fixierposition. Dabei ist das chirurgische Instrument 1 mittels des Fixierelements 102 an dem Probeimplantat 200 fixiert. Durch Verstellen des Fixierelements 102 in seine Freigabeposition lässt sich das chirurgische Instrument 1 von dem Probeimplantat 200 in der anterioren Richtung A trennen.

Die Befestigungseinrichtung 100 weist außerdem wenigstens ein Federelement 106 auf. Bei dem Federelement 106 handelt es sich vorliegend um ein Torsionsfederelement 104. Das Federelement 106 dient zum Erzeugen einer Federkraft, die das Fixierelement 102 in Richtung seiner Fixierposition zwingt. Das Fixierelement 102 ist also mittels der Federkraft in seiner Fixierposition gehalten. Ein Verstellen des Fixierelements 102 in seine Freigabeposition erfolgt unter Überwindung der Federkraft. Infolge der Federkraft kann das Fixierelement 102 beim Einstecken der Positionierstifte 101 selbsttätig an dem Probeimplantat 200 formschlüssig einrasten. Mit anderen Worten: Die Federkraft bewirkt vorliegend, dass das Fixierelement 102 beim Einstecken der Positionierstifte 101 selbsttätig an dem Probeimplantat 200 einrastet.

Die Positionierstifte 101 sind jeweils zwischen einem ersten und einem zweiten Ende 107, 108 anteroposterior längserstreckt. Dabei ist an dem ersten Ende 107 ein erster Endabschnitt 109 vorhanden. Der erste Endabschnitt 109 ist in einer zugehörigen der Aufnahmebohrungen 201 des Probeimplantats 200 unter Ausbildung einer Steckverbindung V in posteriorer Richtung P aufnehmbar. Wenigstens einer der Positionierstifte 101 und seine zugehörige Aufnahmebohrung 201 können in der Art einer engen Spielpassung oder einer Übergangspassung aufeinander abgestimmt sein. Sind einer der Positionierstifte 101 und seine zugehörige Aufnahmebohrung 201 passgenau aufeinander abgestimmt, können der jeweils andere Positionierstift 101 und seine zugehörige Aufnahmebohrung 201 ein mediolaterales Spiel aufweisen. Hierzu kann die betreffende Aufnahmebohrung 201 zur Erzeugung des mediolateralen Spiels langlochartig ausgebildet sein. Vorliegend weisen die Positionierstifte 201 wenigstens im Bereich ihres ersten Endabschnitts 109 einen kreisförmigen Querschnitt auf. Es versteht sich, dass alternativ auch andere Querschnittsformen denkbar sind, beispielsweise in Form eines Ovals oder eines Rechtecks oder eines anderen Vielecks.

Der Formschlussabschnitt 103 bildet vorliegend bezüglich der anterioren Richtung A eine Hinterschneidung 110 aus. Dabei liegen die ersten Enden 107 posterior frei. Die Hinterschneidung 110 liegt anterior frei. Die ersten Enden 107 und die Hinterschneidung 110 sind anteroposterior zueinander entgegengesetzt orientiert. Folglich ist das Probeimplantat 200 zwischen den ersten Enden 107 einerseits und der Hinterschneidung 110 andererseits anteroposterior positionierbar. Vorliegend ist das Probeimplantat 200 zwischen den ersten Enden 107 und der Hinterschneidung 110 eingespannt. In der Einspannung kann die Hinterschneidung 110 ein Widerlager für die Positionierstifte 101 ausbilden.

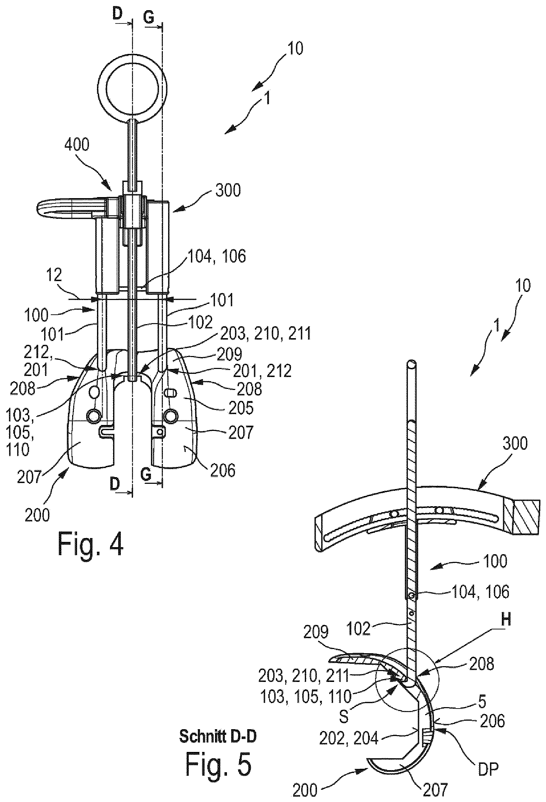

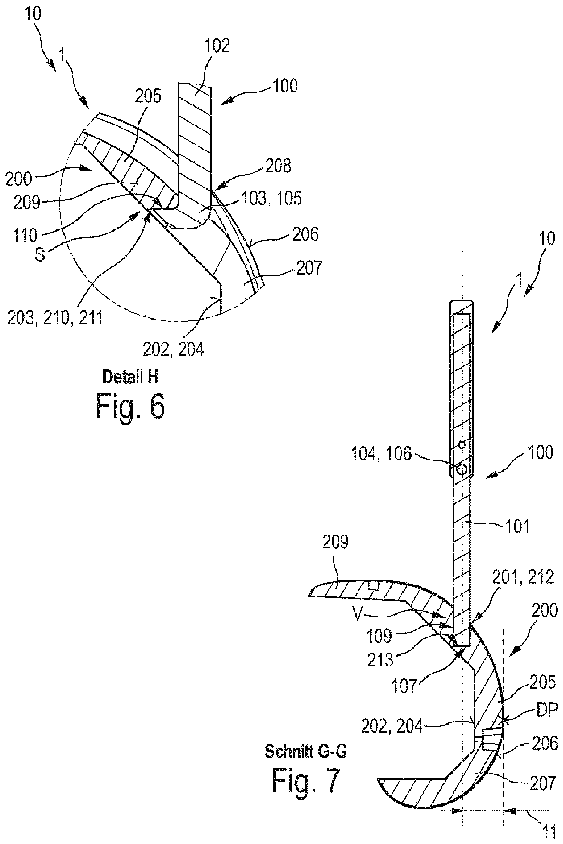

Die Fig. 1 bis 8 zeigen ferner eine Ausführungsform eines erfindungsgemäßen femoralen Probeimplantats 200. Das femorale Probeimplantat 200 ist zur Verwendung bei einer Kniegelenkersatzoperation vorgesehen. Es weist eine Knochenkontaktfläche 202 auf. Die Knochenkontaktfläche 202 ist zur Kontaktierung des distalen Femurs F eingerichtet. Ferner umfasst das femorale Probeimplantat 200 die beiden Aufnahmebohrungen 201. Diese sind jeweils zur Aufnahme eines der Positionierstifte 101 des chirurgischen Instruments 1 eingerichtet. Das femorale Probeimplantat 200 umfasst außerdem einen Fixierabschnitt 203. Der Fixierabschnitt 203 ist zur formschlüssigen Verbindung mit dem Fixierelement 102 des chirurgischen Instruments 1 eingerichtet. Vorliegend weist das femorale Probeimplantat 200 einen, insbesondere klauenförmigen, Grundkörper 205 auf. Die Knochenkontaktfläche 202 ist an einer proximalen Rückseite 204 des Grundkörpers 205 angeordnet. Die proximale Rückseite 204 liegt einer distalen Vorderseite 206 des Grundkörpers 205 gegenüber. Der Grundkörper 205 umfasst zwei mediolateral zueinander beabstandete Kondylenabschnitte 207. Die beiden Kondylenabschnitte 207 sind jeweils anteroposterior erstreckt. Die Kondylenabschnitte 207 gehen an ihren anterioren Enden 202 in einen Verbindungsabschnitt 209 des Grundkörpers 205 über. Die Kondylenabschnitte 207 sind also mittels des Verbindungsabschnitts 209 miteinander verbunden. Dabei ist der Fixierabschnitt 203 an einem posterioren Ende 210 des Verbindungsabschnitts 209 angeordnet. Der Fixierabschnitt 203 befindet sich mediolateral zwischen den beiden Kondylenabschnitten 207. Der Fixierabschnitt 203 befindet sich vorliegend mediolateral zwischen den beiden Aufnahmebohrungen 201. Jeweils eine der Aufnahmebohrungen 201 kann im Bereich eines der Kondylenabschnitte 207 vorhanden sein. Alternativ oder zusätzlich ist der Fixierabschnitt 203 proximodistal im Abstand zu den beiden Aufnahmebohrungen 201 angeordnet. Vorliegend ist der Fixierabschnitt 203 sowohl mediolateral zwischen den beiden Aufnahmebohrungen 201 als auch proximodistal im Abstand zu den beiden Aufnahmebohrungen 201 angeordnet. Der Fixierabschnitt 203 bildet eine Hinterschneidung 211 bezüglich einer posterioren Richtung P aus. Die Hinterschneidung 110 des Fixierelements 102 ist also komplementär ausgebildet zur Hinterschneidung 211 des Fixierabschnitts 203.

Wenigstens eine der Aufnahmebohrungen 201 ist vorliegend als Sackloch 212 ausgebildet. Vorliegend sind beide der Aufnahmebohrungen 201 jeweils als Sackloch 212 ausgebildet. Ein Boden 213 des Sacklochs 212 fungiert als posteriorer Anschlag für die Positionierstifte 101. Entsprechend legt der Boden 213 zusammen mit den ersten Enden 107 der Positionierstifte 101 eine anteroposteriore Position des chirurgischen Instruments 1 relativ zum Probeimplantat 200 fest.

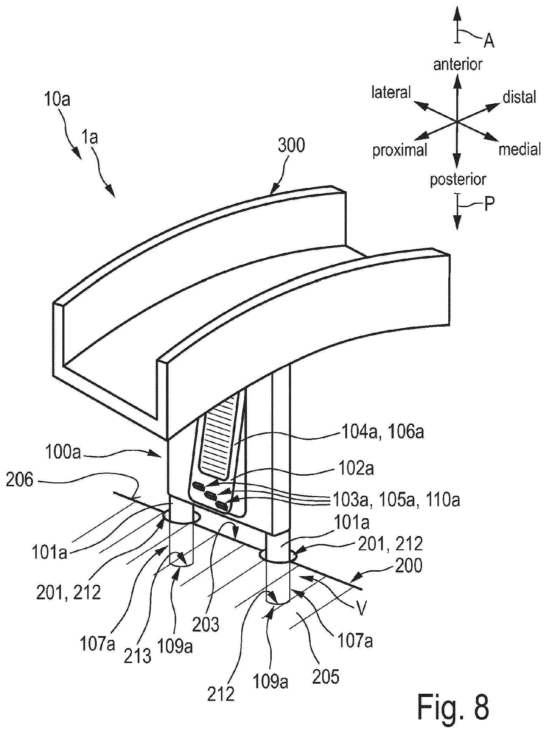

Die weitere Ausführungsform des chirurgischen Instruments 1a gemäß Fig. 8 unterscheidet sich von der Ausführungsform gemäß den Fig. 1 bis 7 in der Ausgestaltung des Fixierelements 102a. Dieses umfasst statt der Torsionsfeder 104 eine Balkenfeder 104a. Die Balkenfeder 104a ist zungenförmig ausgebildet. Ferner sind gemäß Fig. 8 mehrere - genauer drei - Formschlussabschnitte 103a vorhanden. Die Formschlussabschnitte 103a sind als Schnapphaken 105a ausgebildet. Jeder der Formschlussabschnitte 103a bildet eine Hinterschneidung 110a bezüglich der anterioren Richtung A aus. Mittels der Formschlussabschnitte 103a lassen sich drei Raststellungen des chirurgischen Instruments 1a festlegen, in welchen die Befestigungseinrichtung 100a am Probeimplantat 200 anteroposterior einrasten kann. Im Übrigen wird für die Ausführungsform gemäß Fig. 8 auf die vorangehende Beschreibung der Ausführungsform gemäß den Fig. 1 bis 7 verwiesen, wobei die entsprechenden Bezugszeichen in der Fig. 8 um den Kleinbuchstaben a ergänzt sind.

Wie bereits angesprochen, umfasst das chirurgische Instrumentensystem 10, 10a das chirurgisches Instrument 1, 1a sowie das femorale Probeimplantat 200. Das chirurgische Instrument 1, 1a ist lösbar an dem Probeimplantat 200 befestigt. Die Positionierstifte 101, 101a und die Aufnahmebohrungen 201 sind komplementär zueinander ausgebildet. Die Positionierstifte 101, 101a sind in der posterioren Richtung P wenigstens teilweise in die Aufnahmebohrungen 201 eingesteckt. Dabei sind das Fixierelement 102, 102a und der Fixierabschnitt 203 zum Ausbilden des Formschlusses S aufeinander abgestimmt. Das chirurgische Instrument 1, 1a ist an dem Probeimplantat 200 mittels des Formschlusses S gehalten. Der Formschluss S hält das chirurgische Instrument 1, 1a in anteriorer Richtung A an dem Probeimplantat 200. Zwischen den Böden 213 der Sacklochbohrungen 212 und den ersten Enden 107 der Positionierstifte 101 kann ein Formschluss ausgebildet sein, der das chirurgische Instrument 1, 1a in posteriorer Richtung P an dem Probeimplantat 200 hält. Zwischen den Positionierstiften 101 und den zugehörigen Aufnahmebohrungen 201 kann außerdem ein Formschluss ausgebildet sein, der das chirurgische Instrument 100, 100a proximodistal und mediolateral an dem Probeimplantat 200 hält.

Das chirurgische Instrumentensystem 10, 10a umfasst mehrere Probeimplantate 200. Die Probeimplantate 200 unterscheiden sich hinsichtlich ihrer Größe. Die verschieden großen Probeimplantate 200 sind auf Femurknochen unterschiedlicher Größe abgestimmt. Je nach Anatomie des behandelten Patienten lässt sich also ein geeignetes Probeimplantat 200 auswählen. Bei allen Probeimplantaten 200 sind die beiden Aufnahmebohrungen 201 in einem proximodistalen Abstand 11 zu einem proximal äußersten Punkt DP des jeweiligen Probeimplantats 200 angeordnet. Bei diesem Punkt DP kann es sich um einen sogenannten Dwell-Point handeln. Der Abstand 11 ist vorliegend auf die Mittellängsachsen der Aufnahmebohrungen 201 bezogen. Der proximodistale Abstand 11 ist bei allen der Probeimplantate 200 gleich groß, d. h. identisch. Die Aufnahmebohrungen 201 jedes der Probeimplantate 200 sind ferner in einem mediolateralen Abstand 12 voneinander angeordnet. Der mediolaterale Abstand 12 ist beispielsweise auf die Mittellängsachsen der Aufnahmebohrungen 201 bezogen. Der mediolaterale Abstand 12 ist für alle Probeimplantate 200 gleich groß, d. h. identisch. Mit anderen Worten: Unabhängig von ihrer Größe haben alle Probeimplantate 200 des chirurgischen Instrumentensystems 10, 10a Aufnahmebohrungen 201 mit einheitlichem proximodistalem Abstand 11 und einheitlichem mediolateralem Abstand 12.

Das chirurgische Instrumentensystem 10, 10a kann außerdem eine Führungseinrichtung 300 umfassen. Die Führungseinrichtung 300 kann lösbar mit der Befestigungseinrichtung 100 verbunden sein. Mit der Führungseinrichtung 300 kann ferner eine weitere Befestigungseinrichtung lösbar verbunden sein, an welcher ein Tibiaschnittblock zur Führung eines Resektionsschnitts an der proximalen Tibia befestigbar ist. Mittels der Führungseinrichtung 300 kann die weitere Befestigungseinrichtung relativ zur ersten Befestigungseinrichtung 100, 100a ausgerichtet werden. Die lösbare Verbindung der ersten Befestigungseinrichtung 100, 100a mit der Führungseinrichtung 300 kann mittels einer Steckverbindung 400 realisiert sein.

Die Führungseinrichtung 300 kann kreisbogenförmig ausgebildet sein, so dass die Befestigungseinrichtung 100, 100a relativ zur weiteren Befestigungseinrichtung geführt schwenkbeweglich ist. Hierdurch kann die Neigung des Tibiaschnittblocks in einer Führungsebene eingestellt werden. Die Führungsebene ist sagittal ausgerichtet und folglich anteroposterior sowie proximodistal erstreckt. Vorzugsweise schneidet eine Schwenkachse, um welche die beiden Befestigungseinrichtungen relativ zueinander mittels der Führungseinrichtung schwenkbeweglich geführt sind, eine mechanische Tibiaachse in der sagittalen Führungsebene. Die besagte Neigung wird auch als posteriorer oder anteriorer Slope bezeichnet. Die Einstellbarkeit der Neigung des Tibiaschnittblocks - nachfolgend kurz Slope - erlaubt zum einen eine Anpassung an präoperativ festgelegte Parameter. Zum anderen kann eine tatsächlich vorherrschende Flexions- oder Extensionsstellung des Beins durch die Beweglichkeit der Führungseinrichtung berücksichtigt und ausgeglichen werden. Hierdurch lässt sich sicherstellen, dass eine Einstellung des Slopes nicht gleichzeitig zu einer ungewollten Veränderung einer proximodistalen Position des Tibiaschnittblocks und damit einer sogenannten Tibiaschnitthöhe führt.

## Patentansprüche

1. Chirurgisches Instrument (1, 1a) zur Verwendung bei einer Kniegelenkersatzoperation, aufweisend
eine Befestigungseinrichtung (100, 100a), welche zur lösbaren Befestigung an einem an einem distalen Femur (F) angebrachten femoralen Probeimplantat (200) eingerichtet ist,
wobei die Befestigungseinrichtung (100, 100a) zwei mediolateral voneinander beabstandet angeordnete Positionierstifte (101, 101a) aufweist, die zur Positionierung der Befestigungseinrichtung (100, 100a) in zwei zu den Positionierstiften (101, 101a) komplementären Aufnahmebohrungen (201, 201a) des Probeimplantats (200, 200a) in einer posterioren Richtung (P) einsteckbar ausgebildet sind,
wobei die Befestigungseinrichtung (100, 100a) wenigstens ein Fixierelement (102, 102a) zum formschlüssigen Fixieren der Befestigungseinrichtung (100, 100a) an dem Probeimplantat (200) in einer anterioren Richtung (A) aufweist,
wobei die Positionierstifte (101, 101a) jeweils zwischen einem ersten Ende (107, 107a) und einem zweiten Ende (108, 108a) anteroposterior längserstreckt sind,
und wobei ein jeweils am ersten Ende (107, 107a) vorhandener erster Endabschnitt (109, 109a) in einer zugehörigen der Aufnahmebohrungen (201) des Probeimplantats (200) unter Ausbildung einer Steckverbindung (V) in posteriorer Richtung (P) aufnehmbar ist,
**dadurch gekennzeichnet, dass** das Fixierelement (102, 102a) relativ zu den Positionierstiften (101, 101a) zwischen einer Fixier- und einer Freigabeposition verstellbeweglich ist.

2. Chirurgisches Instrument (1, 1a) nach Anspruch 1, **dadurch gekennzeichnet, dass** das Fixierelement (102, 102a) wenigstens einen Formschlussabschnitt (103, 103a) zur Ausbildung eines Formschlusses (S) zwischen der Befestigungseinrichtung (100, 101a) und dem Probeimplantat (200) aufweist.

3. Chirurgisches Instrument (1, 1a) nach Anspruch 1 oder 2, **dadurch gekennzeichnet, dass** das Fixierelement (102, 102a) wenigstens einen Schnapphaken (105, 105a) umfasst.

4. Chirurgisches Instrument (1, 1a) nach einem der vorhergehenden Ansprüche, **dadurch gekennzeichnet, dass** die Befestigungseinrichtung (100, 100a) wenigstens ein Federelement (106, 106a), insbesondere ein Torsions- oder Balkenfederelement (104, 104a), zum Erzeugen einer Federkraft umfasst, die das Fixierelement (102, 102a) in Richtung seiner Fixierposition zwingt.

5. Chirurgisches Instrument (1, 1a) nach Anspruch 2, **dadurch gekennzeichnet, dass** der Formschlussabschnitt (103, 103a) eine Hinterschneidung (110, 110a) bezüglich der anterioren Richtung (A) ausbildet.

6. Chirurgisches Instrument (1, 1a) nach Anspruch 5, **dadurch gekennzeichnet, dass** die ersten Enden (107, 107a) posterior freiliegen und die Hinterschneidung (110, 110a) anterior freiliegt, so dass das Probeimplantat (200) zwischen den ersten Enden (107, 107a) einerseits und der Hinterschneidung (110, 110a) andererseits anteroposterior positionierbar, insbesondere einspannbar, ist.

7. Femorales Probeimplantat (200) zur Verwendung bei einer Kniegelenkersatzoperation, aufweisend
eine Knochenkontaktfläche (202), die zur Kontaktierung eines distalen Femurs (F) eingerichtet ist,
zwei Aufnahmebohrungen (201), die jeweils zur Aufnahme eines Positionierstifts (101, 101a) eines chirurgischen Instruments (1,1a) nach einem der vorhergehenden Ansprüche eingerichtet und anteroposterior längserstreckt sind,
und einen Fixierabschnitt (203), welcher zur formschlüssigen Verbindung mit einem Fixierelement (102, 102a) des chirurgischen Instruments (1, 1a) eingerichtet ist,
wobei die Knochenkontaktfläche (202) an einer proximalen Rückseite (204) eines Grundkörpers (205) des Probeimplantats (200) angeordnet ist und einer distalen Vorderseite (206) des Grundkörpers (205) gegenüberliegt,
und wobei der Grundkörper (205) zwei mediolateral zueinander beabstandete und jeweils anteroposterior erstreckte Kondylenabschnitte (207) aufweist, die an ihren anterioren Enden (202) in einen Verbindungsabschnitt (209) des Grundkörpers (205) übergehen,
**dadurch gekennzeichnet, dass** der Fixierabschnitt (203) an einem posterioren Ende (210) des Verbindungsabschnitts (209) mediolateral zwischen den beiden Kondylenabschnitten (207) angeordnet ist.

8. Femorales Probeimplantat (200) nach Anspruch 7, **dadurch gekennzeichnet**, der Fixierabschnitt (203) mediolateral zwischen den beiden Aufnahmebohrungen (201) oder/und proximodistal im Abstand zu den beiden Aufnahmebohrungen (201) angeordnet ist.

9. Femorales Probeimplantat (200) nach einem der Ansprüche 7 oder 8, **dadurch gekennzeichnet, dass** der Fixierabschnitt (203) eine Hinterschneidung (211) bezüglich einer posterioren Richtung (P) bildet.

10. Femorales Probeimplantat (200) nach einem der Ansprüche 7 bis 9, **dadurch gekennzeichnet, dass** wenigstens eine, vorzugsweise jede, der Aufnahmebohrungen (201) als Sackloch (212) ausgebildet ist.

11. Chirurgisches Instrumentensystem (10, 10a) zur Verwendung bei einer Kniegelenkersatzoperation, aufweisend
ein chirurgisches Instrument (1, 1a) nach einem der Ansprüche 1 bis 6,
ein femorales Probeimplantat (200) nach einem der Ansprüche 7 bis 10, wobei das chirurgische Instrument (1, 1a) lösbar an dem Probeimplantat (200) befestigt ist,
wobei die Positionierstifte (101, 101a) und die Aufnahmebohrungen (201) komplementär zueinander ausgebildet und die Positionierstifte (101, 101a) entlang der posterioren Richtung (P) wenigstens teilweise in die Aufnahmebohrungen (201) gesteckt sind,
wobei das Fixierelement (102, 102a) und der Fixierabschnitt (203) zum Ausbilden eines Formschlusses (S) aufeinander abgestimmt sind und das chirurgische Instrument (1, 1a) an dem Probeimplantat (200) mittels des Formschlusses (S), insbesondere in anteriorer Richtung (A), fixiert ist.

12. Chirurgisches Instrumentensystem (10, 10a) nach Anspruch 11, **dadurch gekennzeichnet, dass** mehrere Probeimplantate (200) unterschiedlicher Größen vorhanden sind, wobei bei allen Probeimplantaten (200) beide der Aufnahmebohrungen (201), insbesondere bezüglich ihrer Mittellängsachsen, in einem identischen proximodistalen Abstand (11) zu einem proximal äußersten Punkt (DP) des jeweiligen Probeimplantats (200) angeordnet sind, wobei die Aufnahmebohrungen (201) jedes der Probeimplantate (200) in einem identischen mediolateralen Abstand (12), insbesondere bezüglich der Mittellängsachsen, voneinander angeordnet sind.

## Claims

1. Surgical instrument (1, 1a) for use in a knee joint replacement operation, having
a fastening device (100, 100a) which is configured for releasably fastening to a femoral trial implant (200) mounted on a distal femur (F),
wherein the fastening device (100, 100a) has two positioning pins (101, 101a) which are arranged spaced apart mediolaterally from each other and which, for the purpose of positioning the fastening device (100, 100a), are designed to be able to be plugged in a posterior direction (P) into two receiving bores (201, 201a), complementary to the positioning pins (101, 101a), of the trial implant (200, 200a),
wherein the fastening device (100, 100a) has at least one fixing element (102, 102a) for fixing the fastening device (100, 100a) in a form-fitting manner to the trial implant (200) in an anterior direction (A),
wherein the positioning pins (101, 101a) are each elongate in an anteroposterior direction between a first and a second end (107, 107a, 108, 108a),
and wherein a first end portion (109, 109a) present at the respective first end (107, 107a) is receivable in the posterior direction (P) in an associated one of the receiving bores (201) of the trial implant (200) so as to form a plug connection (V),
**characterized in that** the fixing element (102, 102a) is adjustably movable, relative to the positioning pins (101, 101a), between a fixing position and a release position.

2. Surgical instrument (1, 1a) according to Claim 1, **characterized in that** the fixing element (102, 102a) has at least one form-fitting portion (103, 103a) for producing a form fit (S) between the fastening device (100, 100a) and the trial implant (200).

3. Surgical instrument (1, 1a) according to Claim 1 or 2, **characterized in that** the fixing element (102, 102a) comprises at least one snap-in hook (105, 105a).

4. Surgical instrument (1, 1a) according to one of the preceding claims, **characterized in that** the fastening device (100, 100a) comprises at least one spring element (106, 106a), in particular a torsion or beam spring element (104, 104a), for generating a spring force which biases the fixing element (102, 102a) in the direction of its fixing position.

5. Surgical instrument (1, 1a) according to Claim 2, **characterized in that** the form-fitting portion (103, 103a) creates an undercut (110, 110a) with respect to the anterior direction (A).

6. Surgical instrument (1, 1a) according to Claim 5, **characterized in that** the first ends (107, 107a) are exposed posteriorly and the undercut (110, 110a) is exposed anteriorly, such that the trial implant (200) can be positioned, in particular clamped, anteroposteriorly between the first ends (107, 107a) on the one hand and the undercut (110, 110a) on the other hand.

7. Femoral trial implant (200) for use in a knee joint replacement operation, having
a bone contact surface (202) which is configured for contacting a distal femur (F),
two receiving bores (201) which are each configured to receive a positioning pin (101, 101a) of a surgical instrument (1, 1a) according to one of the preceding claims and are each elongated anteroposteriorly,
and a fixing portion (203) which is configured for form-fit connection to a fixing element (102, 102a) of the surgical instrument (1, 1a),
wherein the bone contact surface (202) is arranged on a proximal rear face (204) of a main body (205) of the trial implant (200) and lies opposite a distal front face (206) of the main body (205),
wherein the main body (205) has two condyle portions (207) which are spaced mediolaterally apart from each other and which each extend in the anteroposterior direction and transition at their anterior ends (202) into a connection portion (209) of the main body (205),
**characterized in that** the fixing portion (203) is arranged at a posterior end (210) of the connection portion (209), mediolaterally between the two condyle portions (207).

8. Femoral trial implant (200) according to Claim 7, **characterized in that** the fixing portion (203) is arranged mediolaterally between the two receiving bores (201) and/or at a proximodistal distance from the two receiving bores (201).

9. Femoral trial implant (200) according to Claim 7 or 8, **characterized in that** the fixing portion (203) creates an undercut (211) with respect to a posterior direction (P).

10. Femoral trial implant (200) according to one of Claims 7 to 9, **characterized in that** at least one of the receiving bores (201), preferably each of them, is designed as a blind hole (212).

11. Surgical instrument system (10, 10a) for use in a knee joint replacement operation, having
a surgical instrument (1, 1a) according to one of Claims 1 to 6,
a femoral trial implant (200) according to one of Claims 7 to 10, wherein the surgical instrument (1, 1a) is fastened releasably to the trial implant (200),
wherein the positioning pins (101, 101a) and the receiving bores (201) are designed complementing each other, and the positioning pins (101, 101a) are at least partially plugged into the receiving bores (201) along the posterior direction (P),
wherein the fixing element (102, 102a) and the fixing portion (203) are tailored to each other to produce a form fit (S), and the surgical instrument (1, 1a) is fixed on the trial implant (200) by means of the form fit (S), in particular in the anterior direction (A).

12. Surgical instrument system (10, 10a) according to Claim 11, **characterized in that** a plurality of femoral trial implants (200) of different sizes are present, wherein, in all of the femoral trial implants (200), both of the receiving bores (201) are arranged, particularly with respect to their central longitudinal axes, at an identical proximodistal distance (11) from a proximally outermost point (DP) of the respective femoral trial implant (200), wherein the receiving bores (201) of each of the femoral trial implants (200) are arranged at an identical mediolateral distance (12) from each other, in particular with respect to the central longitudinal axes.

## Revendications

1. Instrument chirurgical (1, 1a) destiné à être utilisé lors d'une opération de remplacement de l'articulation du genou, comprenant
un dispositif de fixation (100, 100a) qui est conçu pour être fixé de manière amovible à un implant fémoral d'essai (200) fixé à un fémur distal (F),
le dispositif de fixation (100, 100a) comprenant deux broches de positionnement (101, 101a) agencées de manière espacée l'une de l'autre dans le sens médio-latéral, qui sont configurées insérables dans une direction postérieure (P) pour le positionnement du dispositif de fixation (100, 100a) dans deux alésages de réception (201, 201a) de l'implant d'essai (200, 200a) complémentaires aux broches de positionnement (101, 101a),
le dispositif de fixation (100, 100a) comprenant au moins un élément de fixation (102, 102a) pour la fixation par complémentarité de forme du dispositif de fixation (100, 100a) sur l'implant d'essai (200) dans une direction antérieure (A),
les broches de positionnement (101, 101a) s'étendant chacune dans la direction antéro-postérieure entre une première extrémité (107, 107a) et une deuxième extrémité (108, 108a),
et un premier segment d'extrémité (109, 109a) présent à chaque première extrémité (107, 107a) étant apte à être reçu dans un alésage de réception correspondant (201) de l'implant d'essai (200) en formant une liaison par insertion (V) dans la direction postérieure (P),
**caractérisé en ce que** l'élément de fixation (102, 102a) est apte à être déplacé par rapport aux broches de positionnement (101, 101a) entre une position de fixation et une position de libération.

2. Instrument chirurgical (1, 1a) selon la revendication 1, **caractérisé en ce que** l'élément de fixation (102, 102a) présente au moins une section à engagement par complémentarité de forme (103, 103a) pour former un engagement par complémentarité de forme (S) entre le dispositif de fixation (100, 101a) et l'implant d'essai (200).

3. Instrument chirurgical (1, 1a) selon la revendication 1 ou la revendication 2, **caractérisé en ce que** l'élément de fixation (102, 102a) comprend au moins un crochet à encliquetage (105, 105a).

4. Instrument chirurgical (1, 1a) selon l'une des revendications précédentes, **caractérisé en ce que** le dispositif de fixation (100, 100a) comprend au moins un élément élastique (106, 106a), en particulier un élément élastique à torsion ou à lame (104, 104a) destiné à générer une force élastique qui force l'élément de fixation (102, 102a) dans la direction de sa position de fixation.

5. Instrument chirurgical (1, 1a) selon la revendication 2, **caractérisé en ce que** la partie de liaison par complémentarité de forme (103, 103a) forme une contre-dépouille (110, 110a) par rapport à la direction antérieure (A).

6. Instrument chirurgical (1, 1a) selon la revendication 5, **caractérisé en ce que** les premières extrémités (107, 107a) sont dégagées postérieurement et la contre-dépouille (110, 110a) est dégagée antérieurement, de sorte que l'implant d'essai (200) est apte à être positionné, en particulier serré, dans la direction antéro-postérieure, entre les premières extrémités (107, 107a) d'une part et la contre-dépouille (110, 110a) d'autre part.

7. Implant fémoral d'essai (200) destiné à être utilisé dans une opération de remplacement de l'articulation du genou, comprenant
une surface (202) de contact avec l'os qui est conçue pour être en contact avec un fémur distal (F),
deux alésages de réception (201) qui sont chacun conçus pour recevoir une broche de positionnement (101, 101a) d'un instrument chirurgical (1, 1a) selon l'une des revendications précédentes et qui s'étendent longitudinalement dans la direction antéro-postérieure,
et une partie de fixation (203) qui est conçue pour être reliée par complémentarité de forme à un élément de fixation (102, 102a) de l'instrument chirurgical (1, 1a), la surface (202) de contact avec l'os étant agencée sur une face arrière proximale (204) d'un corps de base (205) de l'implant d'essai (200) et faisant face à une face avant distale (206) du corps de base (205),
et le corps de base (205) comprenant deux parties de condyle (207) espacées l'une de l'autre dans la direction médio-latérale et s'étendant chacune dans la direction antéro-postérieure, qui, à leurs extrémités antérieures (202) se fondent dans une partie de liaison (209) du corps de base (205),
**caractérisé en ce que** la partie de fixation (203) est agencée à une extrémité postérieure (210) de la partie de liaison (209) dans la direction médio-latérale entre les deux parties de condyle (207).

8. Implant fémoral d'essai (200) selon la revendication 7, **caractérisé en ce que** la partie de fixation (203) est agencée médio-latéralement entre les deux alésages de réception (201) ou/et proximodistalement à distance des deux alésages de réception (201).

9. Implant fémoral d'essai (200) selon l'une des revendications 7 et 8, **caractérisé en ce que** la partie de fixation (203) forme une contre-dépouille (211) par rapport à une direction postérieure (P).

10. Implant fémoral d'essai (200) selon l'une des revendications 7 à 9, **caractérisé en ce qu'**au moins un, de préférence chacun, des alésages de réception (201) est réalisé sous forme de trou borgne (212).

11. Système d'instruments chirurgicaux (10, 10a) destiné à être utilisé lors d'une opération de remplacement de l'articulation du genou, comprenant
un instrument chirurgical (1, 1a) selon l'une des revendications 1 à 6,
un implant fémoral d'essai (200) selon l'une des revendications 7 à 10, l'instrument chirurgical (1, 1a) étant fixé de manière amovible à l'implant d'essai (200), les broches de positionnement (101, 101a) et les alésages de réception (201) étant réalisés de manière complémentaire les uns aux autres, et les broches de positionnement (101, 101a) étant insérées au moins partiellement dans les alésages de réception (201) le long de la direction postérieure (P),
l'élément de fixation (102, 102a) et la partie de fixation (203) étant adaptés l'un à l'autre pour former une liaison par complémentarité de forme (S), et
l'instrument chirurgical (1, 1a) étant fixé à l'implant d'essai (200) au moyen de la liaison par complémentarité de forme (S), en particulier dans la direction antérieure (A).

12. Système d'instruments chirurgicaux (10, 10a) selon la revendication 11, **caractérisé en ce qu'**il existe plusieurs implants d'essai (200) de tailles différentes, les deux alésages de réception (201) de tous les implants d'essai (200) étant agencés, en particulier par rapport à leurs axes longitudinaux centraux, à une distance proximo-distale (11) identique par rapport à un point proximal le plus extérieur (DP) de l'implant d'essai (200) respectif, les alésages de réception (201) de chacun des implants d'essai (200) étant agencés à une distance médio-latérale (12) identique les uns par rapport aux autres, en particulier par rapport aux axes longitudinaux médians.
